(19) Europäisches Patentamt
European Patent Office
Office européen des brevets

(11) **EP 3 591 664 A1**

(12) **EUROPEAN PATENT APPLICATION**

(43) Date of publication:
**08.01.2020 Bulletin 2020/02**

(51) Int Cl.:
**G16H 50/30** (2018.01)    **A61B 5/16** (2006.01)
**A61B 3/113** (2006.01)

(21) Application number: **18182000.2**

(22) Date of filing: **05.07.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Université Libre de Bruxelles (ULB)
1050 Bruxelles (BE)**

(72) Inventors:
• **Kissine, Mikhail
  1190 Forest (BE)**
• **Deliens, Gaétane
  1800 Vilvoorde (BE)**

(74) Representative: **ABYOO
Centre Monnet
Avenue Jean Monnet 1
1348 Louvain-La-Neuve (BE)**

(54) **METHOD FOR EVALUATING A RISK OF NEURODEVELOPMENTAL DISORDER WITH A CHILD**

(57)     A method is provided which performs at least two different tests on a given child (20) at respectively at least two different ages of said child. In the course of a first test, a first information is displayed to the child and meanwhile a controller (15) acquires first data about eye movements of the child via an eye-tracking device (10) and determines a first score by comparison of said first data with first pre-recorded reference data. The same is performed in the course of a second test at a second age of said child with second information which is different from the first information, hence yielding a second score. A composite score is then calculated for said child in function of at least the first and the second scores. This composite score is a more reliable indication of a risk of neurodevelopmental disorder than indicators obtained with known methods.

Fig. 6

EP 3 591 664 A1

**Description**

**Field of the invention**

The invention relates to methods and devices for evaluating a risk of a neurodevelopmental disorder - more particularly of an Autism Spectrum Disorder (ASD) - with children.

**Description of prior art**

International patent publication WO 2015/057321 discloses a method which, though fundamentally different, is in a field which is close to the field of the invention.

According to this known method, first eye movement data of a child is collected in the course of a first session during which a first visual stimulus is shown to the child and second eye movement data of the same child is collected in the course of a second session during which a second visual stimulus is shown to the child. The first and second eye movement data are then compared with each other to identify a change in visual fixation by the child on the two visual stimuli. A decline in visual fixation is described as being a marker of a developmental, cognitive, social or mental disability or ability of the child. This method, which is thus based on an evolutionary curve of fixations, can lead to significant differences at the level of a group performance, but it is not sensitive enough to allow the establishment of individual thresholds.

International patent publication WO2014/164858 discloses a method which, though fundamentally different, is also in a field which is close to the field of the invention. According to this known method, visual information is shown to a patient and fixations of the patient with respect to this information is analyzed and compared in order to calibrate the system and later to verify its accuracy when in operation.

**Summary of the invention**

It is an object of the invention to provide a method and device which deliver more accurate results for individuals, more particularly results with less false positive results and/or less false negative results.

The invention is defined by the independent claims. The dependent claims define advantageous embodiments.

According to the invention, there is provided a method for evaluating a risk of neurodevelopmental disorder with a child, the method comprising the steps of:

- providing a machine comprising a display screen, an eye-tracking device, a controller configured for displaying information on the display screen and for reading and recording eye position data from the eye-tracking device;
- having the child passing a first test in the course of a first time period, the first test comprising, in sequence:

  - having the child looking at the display screen and directing the eye-tracking device towards at least one eye of said child,
  - displaying first information on the display screen and meanwhile reading and recording eye position data from the eye-tracking device, thereby obtaining first recorded movements of the at least one eye of the child,
  - comparing data from the first recorded movement of the at least one eye of the child with first pre-recorded reference data and calculating a first score in function of said comparison;
  - having the child passing at least a second test in the course of a second time period, the second test comprising, in sequence:

    - having the child looking at the display screen and directing the eye-tracking device towards at least one eye of said child,
    - displaying second information on the display screen and meanwhile reading and recording eye position data from the eye-tracking device, thereby obtaining second recorded movements of the at least one eye of the child,
    - comparing data from the second recorded movement of the at least one eye of the child with second pre-recorded reference data and calculating a second score in function of said comparison;

    characterized in that

    - the second time period starts at least 24 hours later than the end of first time period,
    - the second information is different from the first information, and
    - the controller calculates and delivers a composite score which is a function of at least the first score and the second score.

Such a method is more reliable than the known methods because it checks at least two different developmental aspects at two different ages of the child or at an age at which the child should normally have acquired the two different development aspects, so that each test of each aspect can be adapted to the specific development stage of the child. Combining the at least two results obtained into a composite score further improves the reliability of the method.

It is to be stressed that the composite score resulting from the method according to the invention is in only indicative of a risk of a neurodevelopmental disorder with the child to whom it is applied and that it does neither provide a diagnostic per se of such a disorder nor does it presuppose in any way of a later diagnostic of such disorder.

Preferably, the neurodevelopmental disorder is an Autism Spectrum Disorder.

Preferably, the first time period is a within a period during which the child has an age comprised between 4 and 10 months, preferably between 6 and 8 months, and the second time period is within a period during which the child has an age comprised between 7 and 13 months, preferably between 9 and 11 months.

A child reaches indeed typically two different development stages during respectively these two specific time periods.

More preferably, the method further comprises the steps of:

- having the child passing a third test in the course of a third time period, the third test comprising, in sequence:

  - having the child looking at the display screen and directing the eye-tracking device towards at least one eye of said child,
  - displaying third information on the display screen and meanwhile reading and recording eye position data from the eye-tracking device, thereby obtaining third recorded movements of the at least one eye of the child,
  - comparing data from the third recorded movement of the at least one eye of the child with third pre-recorded reference data and calculating a third score in function of said comparison;

  wherein

  - the third time period starts at least 24 hours later than the end of second time period,
  - the third information is different from the first and from the second information, and
  - the controller calculates and delivers a composite score which is a function of at least the first score, the second score and the third score.

Adding a third test and performing it after the second test with information which is different from the first and from the second information, increases the reliability of the method. It allows indeed to test and to take into account a different (third) development aspect at a different (third) development stage of the child.

More preferably, the third time period is a within a period during which the child has an age comprised between 10 and 16 months, preferably between 12 and 14 months.

A child reaches indeed typically a third development stage during this specific third time period.

The invention also provides an apparatus for evaluating a risk of neurodevelopmental disorder with a child, said apparatus comprising a display screen, an eye-tracking device and a controller, said controller being configured for:

in the course of a first time period:

- displaying first information on the display screen and meanwhile reading and recording eye position data from the eye-tracking device while the child is looking at the display screen, thereby obtaining first recorded movements of at least one eye of the child, and
- comparing data from the first recorded movement of the at least one eye of the child with first pre-recorded reference data and calculating a first score in function of said comparison, and

in the course of a second time period:

- displaying second information on the display screen and meanwhile reading and recording eye position data from the eye-tracking device while the child is looking at the display screen, thereby obtaining second recorded movements of at least one eye of the child, and
- comparing data from the second recorded movement of the at least one eye of the child with second pre-recorded reference data and calculating a second score in function of said comparison,

characterized in that

- the second time period starts at least 24 hours later than the end of first time period,
- the second information is different from the first information, and
- the controller is configured to calculate and deliver a composite score which is a function of at least the first score and the second score.

**Short description of the drawings**

These and further aspects of the invention will be explained in greater detail by way of examples and with reference to the accompanying drawings in which:

Fig.1　shows a flowchart of an exemplary method according to the invention;
Fig.2　shows a series of exemplary display screens that are shown to a child during the first test;
Fig.3　shows a series of exemplary display screens that are shown to a child during the second test;
Fig.4　shows a series of exemplary display screens that are shown to a child during the third test;
Fig.5　shows a flowchart of an exemplary preferred method according to the invention;
Fig.6　schematically shows an apparatus according to the invention;

The drawings of the figures are neither drawn to scale nor proportioned. Generally, similar or identical components are denoted by the same reference numerals in the figures.

**Detailed description of embodiments of the invention**

According to the invention, there is provided a method for evaluating a risk of neurodevelopmental disorder with a child.
The method first comprises the step of providing a machine comprising a display screen (5), an eye-tracking device (10), a controller (15) configured for displaying information on the display screen (5) and for reading and recording eye position data from the eye-tracking device (10).
The machine may for example be a portable personal computer or a tablet computer to which an eye tracking device is connected. Various commercially available eye tracking devices may be used, such as for example an Eye Tracker 4C model from Tobii Technology or a RED-m from SensoMotoric Instruments or a RK-464 model from ISCAN Inc. or an TM5 Mini from EyeTech. These eye-tracking devices come with appropriate control software which, once installed and run, enable the controller (15) (which is generally the computer itself) to read and record eye position data from the eye-tracking device (10). The machine may for example also be a portable personal computer or a tablet computer with a built-in eye tracking device and corresponding control software. The display screen (5) may be an individual video screen or a video screen integrated into another device such as a personal computer or a tablet computer or a video projector or any other electronic means for displaying images. The controller (15) may be a dedicated controller (15) or a personal computer or a tablet computer or any other means having the capability to display information on the display screen (5), to read and record eye position data from the eye-tracking device (10), to perform operations on these data, to read data from a data storage medium and to save results on a data storage medium.
Once the machine is set-up, a child to be examined passes a first test in the course of a first time period.
The first test comprises the following substeps, in sequence:

- having the child looking at the display screen (5) and directing the eye-tracking device (10) towards at least one eye of said child,
- displaying first information on the display screen (5) and meanwhile reading and recording eye position data from the eye-tracking device (10), thereby obtaining first recorded movements of the at least one eye of the child,
- comparing data from the first recorded movement of the at least one eye of the child with first pre-recorded reference data and calculating a first score in function of said comparison.

The first pre-recorded reference data may for example be the same or similar eye movement data obtained from a representative sample of children having no neurodevelopmental disorder and who were submitted to the first test.

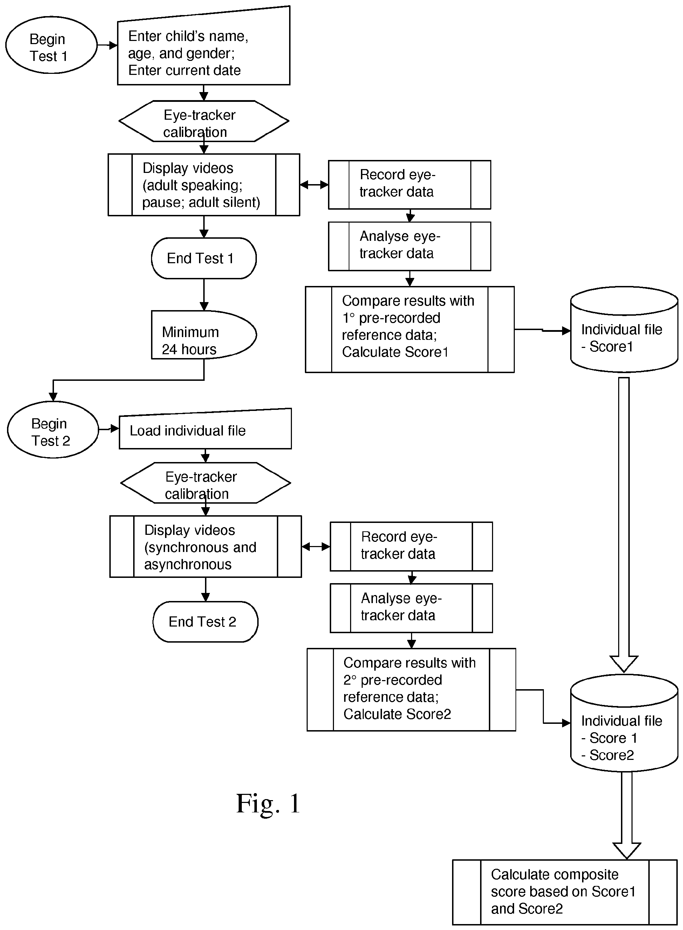

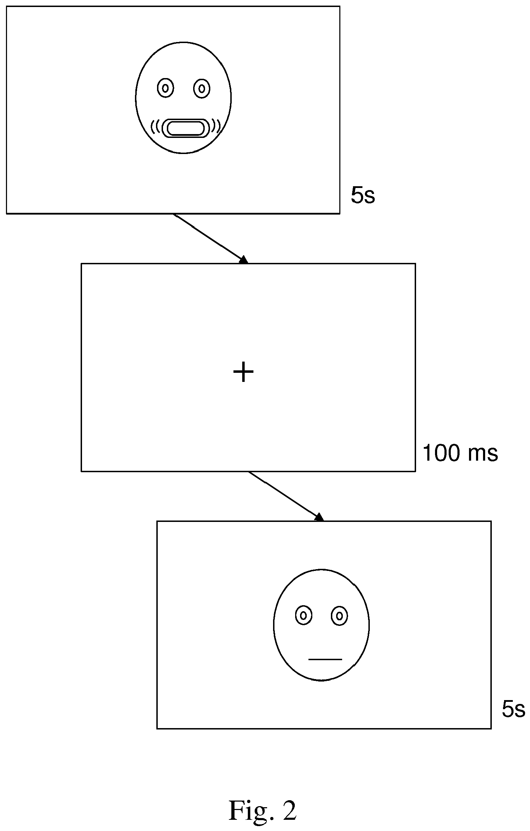

Fig.1 shows a flowchart of an exemplary method according to the invention, which will now be described in more detail. An exemplary first test is a test wherein attention of the child to articulatory movements is evaluated. This first test is preferably performed when the child is between 4 and 10 months old, preferably when he is between 6 and 8 months old. To this end, the machine is switched ON and the child is instructed to look at the display screen (5) while the eye tracking device is oriented towards his eyes. As shown on Fig.2, a video of the face of an adult is then displayed on the display screen (5) with an alternation of phases where the adult is uttering (a) word(s) and phases wherein the adult is silent, preferably with void screens (or screens not showing the adult) in-between these uttering and silent phases.

This video constitutes in this example what we previously called "the first information".

A phase where the adult is uttering (a) word(s) may for example last for 5 s, a phase where the adult is silent may for example last for 5 s, and the display of a void screen (or of a screen not showing the adult) may for example last for 100 ms. In the course of these phases, the controller (15) reads and records data from the eye-tracking device (10) and looks for fixations of the mouth (or mouth region) of the adult by the child while the adult is uttering words and while he is silent. The controller (15) then calculates the following ratio for example:

$$\text{Ratio1.1} = [\text{ total duration of fixations on the mouth (or on the mouth region) during the time segment when the adult is uttering words] / [total duration of fixations on the mouth (or mouth region) during the time segment when the adult is silent].}$$

A fixation corresponds to a situation where it is considered that the eye of the child doesn't move significantly with respect to a target object or location in his visual field, i.e. a phase during which the child continuously looks at said target or location for a minimum duration (all within a given tolerance of course). Most eye-tracking devices include a function of detection of fixations with preset parameters which can generally be modified by the user. The duration of a fixation is therefore the duration of said phase.

Alternatively or additionally, the controller (15) may calculate the following ratio for example:

$$\text{Ratio1.2} = [\text{number of fixations on the mouth while the adult is uttering words] / [number of fixations on the mouth while the adult is silent]}$$

Ratio1.1 and/or Ratio1.2 is (are) then compared to first pre-recorded reference data and a first score is calculated in function of said comparison.

The first pre-recorded reference data may for example be respectively the same ratios obtained when submitting a representative sample of children having no neurodevelopmental disorder to the first test.

The first score may then for example be a function of a difference between two corresponding ratios or between averages of the two corresponding ratios or a mix of both.

For example: First score for the child under test = (Ratio1.1 for the child under test) - (average Ratio. 1.1 for the sample of children having no neurodevelopmental disorder).

The first score may represent a standardized score of attention of the child to the mouth in speech condition.

Here follows in some more detail an exemplary process for obtaining a first score when using Ratio1.2:

1. Set Counter-speak = 0
2. Set Counter-silent = 0
3. Display video1 on the screen (adult is speaking)
4. Detect fixations
5. If no fixation detected, goto step 9, else :
6. Localize each fixation (xy coordinates on the screen)
7. Compare coordinates of each fixation with coordinates of expected fixation (= xy coordinates of the mouth of the speaking adult on the screen)
8. Increase counter-speak by one unit for each fixation if its coordinates match the coordinates of the expected fixation (within tolerance range)
9. Pause
10. Display video2 on the screen (adult is silent)
11. Detect fixations
12. If no fixation detected, goto step 16, else :
13. Localize each fixation (xy coordinates on the screen)
14. Compare coordinates of each fixation with coordinates of expected fixation (= xy coordinates of the mouth of the silent adult on the screen)
15. Increase counter-silent by one unit for each fixation if its coordinates match the coordinates of the expected fixation (within tolerance range)
16. Pause

17. Optionally repeat steps 3 to 16, N times
18. Calculate Ratio1.2 = counter-speak/counter-silent
19. Compare Ratio 1.2 with pre-recorded reference data
20. Calculate first score in function of said comparison

Next, the same child passes at least a second test in the course of a second time period. The second time period starts at least 24 hours later than the end of first time period.
The second test comprises the following substeps, in sequence:

- having the child looking at the display screen (5) and directing the eye-tracking device (10) towards at least one eye of said child,
- displaying second information on the display screen (5), the second information being different from the first information, and meanwhile reading and recording eye position data from the eye-tracking device (10), thereby obtaining second recorded movements of the at least one eye of the child,
- comparing data from the second recorded movement of the at least one eye of the child with second pre-recorded reference data and calculating a second score in function of said comparison.

The second reference data may for example be the same or similar eye movement data obtained from a representative sample of children having no neurodevelopmental disorder and who were submitted to the second test.

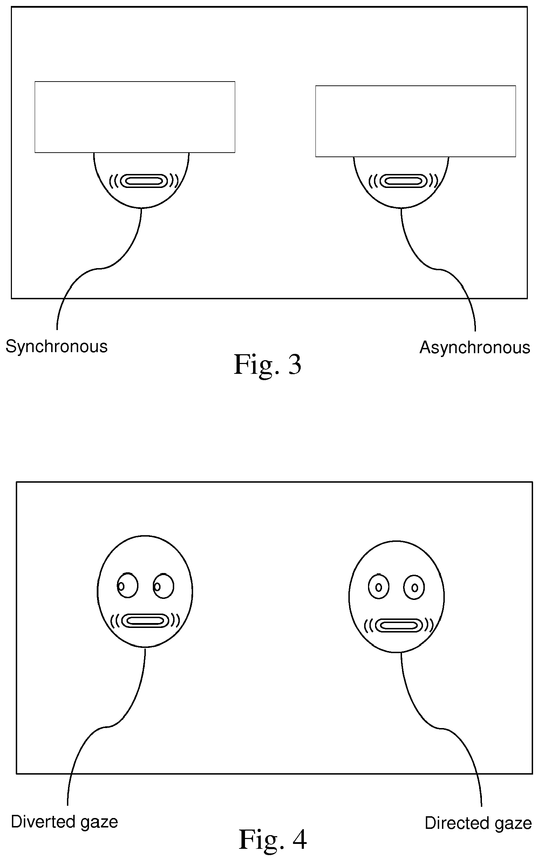

An exemplary second test is a test wherein the capacity of audio-visual integration in speech is evaluated. This second test is preferably performed when the child is between 7 and 13 months old, preferably when he is between 9 and 11 months old. To this end, the machine is switched ON and the child is instructed to look at the display screen (5) while the eye tracking device is oriented towards his eyes. As shown on Fig.3, two videos of the mouth of an adult uttering (a) word(s) or (a) syllable(s) are then displayed simultaneously on two different parts of the display screen (5) (one video for each mouth), for example one video on a left half and one video on a right half of the display screen (5). These two videos together constitute in this example what we previously called "the second information". Meanwhile, a loudspeaker (30) plays the sound corresponding to the uttered word(s) or syllable(s). The loudspeaker (30) may for example be a loudspeaker (30) built in into the machine such as the loudspeaker (30) built into a portable computer or into a tablet computer for example.
The mouth displayed on one part of the display screen (5) (for example the left part) is uttering words or syllables synchronously with the sound played by the loudspeaker (30) (synchronous video) and the mouth displayed on the other part of the display screen (5) (for example the right part) is uttering words or syllables asynchronously or desynchronized with the sound played by the loudspeaker (30) (asynchronous or desynchronized video).
In the course of these displays, the controller (15) reads and records data from the eye-tracking device (10) and looks for fixations by the child on respectively each of the two mouths (or mouth regions) shown on the display screen (5) while the adult is uttering the word(s) or syllable(s). The controller (15) then calculates the following ratio for example:

$$\text{Ratio2.1} = [\text{sum of fixation durations on the mouth (or mouth region) shown on the synchronous video}] / [\text{sum of fixation durations on the mouth (or mouth region) shown on the synchronous video plus sum of fixation durations on the mouth (or mouth region) shown on the asynchronous video}].$$

Alternatively or additionally, the controller (15) may calculate the following ratio for example:

$$\text{Ratio2.2} = [\text{number of fixations on the mouth (or mouth region) shown on the synchronous video}] / [\text{number of fixations on the mouth (or mouth region) shown on the synchronous video plus number of fixations on the mouth (or mouth region) shown on the asynchronous video}].$$

By "mouth region", one means a location in the close vicinity of the mouth, such as for example a location which is not further away from the border of the mouth than 20% of the largest dimension of said mouth when displayed on the display screen (5). If the largest dimension of said mouth when displayed on the display screen (5) is 5 cm for example, then a point located at 6 cm or more from the border of the mouth would not be part of the mouth region whereas a point located at 5,9999 cm or less from the border of the mouth would be part of the mouth region.

Ratio2.1 and/or Ratio 2.2 is (are) then compared to second pre-recorded reference data and a second score is calculated in function of said comparison.

The second pre-recorded reference data may for example be respectively the same ratios obtained when submitting a representative sample of children having no neurodevelopmental disorder to the second test. The second score may then for example be a function of a difference between two corresponding ratios or between averages of the two corresponding ratios or a mix of both. For example: Second score for the child under test = (Ratio2.1 for the child under test) - (average Ratio.2.1 for the sample of children having no neurodevelopmental disorder).

The second score may represent a standardized score of audio-visual integration in speech of the child.

Finally, the controller (15) calculates and delivers a composite score which is a function of at least the first score and the second score.

The composite score may for example be a weighted average of the first score and the second score. The composite score is indicative of a risk that the child has a neurodevelopmental disorder.

Preferably, the first time period is a within a period during which the child has an age comprised between 4 and 10 months, preferably between 6 and 8 months, and the second time period is within a period during which the child has an age comprised between 7 and 13 months, preferably between 9 and 11 months.

Alternatively, the first time period and the second time period are both within a period during which the child has an age comprised between 7 and 13 months, preferably between 9 and 11 months, yet still with at least 24 hours between the end of the first time period and the start of the second time period.

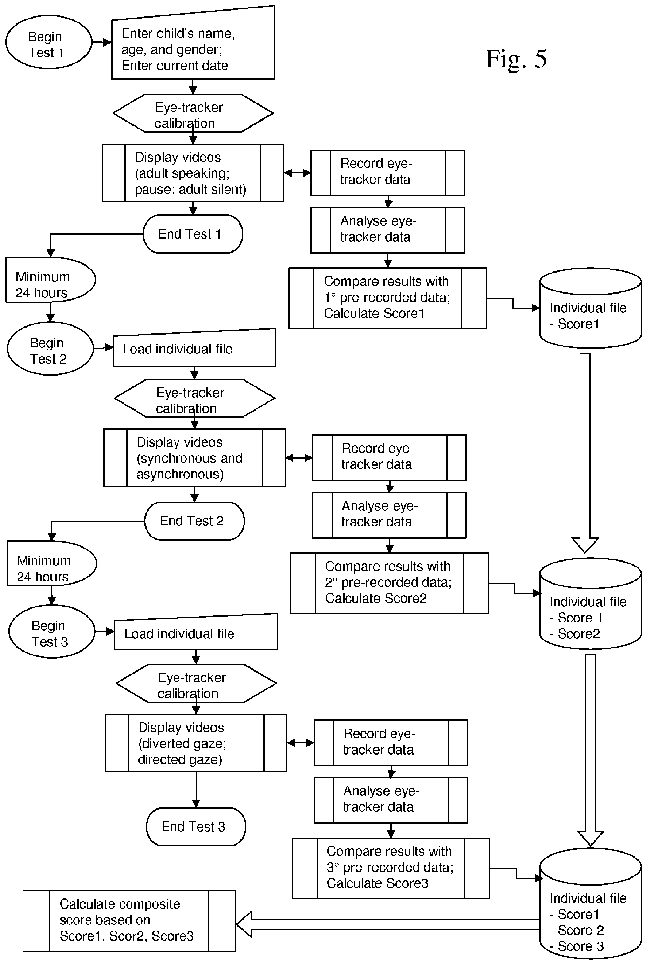

Fig.5 shows a flowchart of an exemplary preferred method according to the invention. Preferably, the method according to the invention further comprises the step of having the child passing a third test in the course of a third time period. The third time period starts at least 24 hours later than the end of the second time period. The third test comprises the following substeps, in sequence:

- having the child looking at the display screen (5) and directing the eye-tracking device (10) towards at least one eye of said child,
- displaying third information on the display screen (5), the third information being different from the first and from the second information, and meanwhile reading and recording eye position data from the eye-tracking device (10), thereby obtaining third recorded movements of the at least one eye of the child,
- comparing data from the third recorded movement of the at least one eye of the child with third pre-recorded reference data and calculating a third score in function of said comparison.

The third reference data may for example be the same or similar eye movement data obtained from a representative sample of children having no neurodevelopmental disorder and who were submitted to the third test.

An exemplary third test is a test wherein the attention to eye-region in speech is evaluated. This third test is preferably performed when the child is between 10 and 16 months old, preferably when he is between 12 and 14 months old. To this end, the machine is switched ON and the child is instructed to look at the display screen (5) while the eye tracking device is oriented towards his eyes. As shown on Fig.4, two videos of the face of an adult uttering (a) word(s) or (a) syllable(s) are then displayed simultaneously on two different parts of the display screen (5) (one video for each face), for example one video on a left half and one video on a right half of the display screen (5). These two videos together constitute in this example what we previously called "the third information".

Meanwhile, a loudspeaker (30) plays the sound corresponding to the uttered word(s) or syllable(s). The loudspeaker (30) may for example be a loudspeaker built in into the machine such as the loudspeaker built into a portable computer or into a tablet computer for example.

The face displayed on one part of the display screen (5) (for example the right part) is oriented towards to child (directed gaze video) and the face displayed on the other part of the display screen (5) (for example the left part) is oriented away from the child (diverted gaze video).

In the course of these displays, the controller (15) reads and records data from the eye-tracking device (10) and looks for fixations by the child on respectively each of the two faces shown on the display screen (5) while the adult is uttering the word(s) or syllable(s).

The controller (15) then calculates the following ratio for example:

Ratio3.1 = [sum of fixation durations on the face shown on the directed gaze video] / [sum of fixation durations on the face shown on the directed gaze video plus sum of fixation durations on the face shown on the diverted gaze video].

Alternatively or additionally, the controller (15) may calculate the following ratio for example:

Ratio3.2 = [number of fixations on the face shown on the directed gaze video] / [number of fixations on the face shown on the directed gaze video plus number of fixations on the face shown on the diverted gaze video].

Ratio3.1 and/or Ration3.2 is (are) then compared to third pre-recorded reference data and a third score is calculated in function of said comparison.

The third pre-recorded reference data may for example be respectively the same ratios obtained when submitting a representative sample of children having no neurodevelopmental disorder to the third test. The third score may then for example be a function of a difference between two corresponding ratios or between averages of the two corresponding ratios or a mix of both.

For example: Third score for the child under test = (Ratio3.1 for the child under test) - (average Ratio3.1 for the sample of children having no neurodevelopmental disorder).

The third score may represent a standardized score of attention to eye-region in speech of the child.

The controller (15) then calculates and delivers a composite score which is a function of at least the first score, the second score and the third score.

The composite score may for example be a weighted average of the first score, the second score and the third score. The composite score is indicative of a risk that the child has a neurodevelopmental disorder.

Preferably, the third time period is a within a period during which the child has an age comprised between 10 and 16 months, preferably between 12 and 14 months.

Alternatively, the first time period, the second time period and the third time period are all within a period during which the child has an age comprised between 10 and 16 months, preferably between 12 and 14 months, yet still with at least 24 hours between the end of the first time period and the start of the second time period and with at least 24 hours between the end of the second time period and the start of the third time period.

Preferably, the method according to the invention further comprises the step of reading (or inputting) and recording identification data of the child under test such as for example his name, birth date, gender, etc..., before starting the first test and the step of linking said data with the first score, with at least the second score and with the composite score obtained for said child. This allows to easily retrieve individual scores based on the identity of the child.

Preferably, the method according to the invention further comprises the step of inputting the current age of the child before starting the first, the second or the third test and of having the controller (15) first checking the inputted age of child and then automatically selecting and executing respectively one of the first, the second or the third test in function of the current age of the child. This reduces the risk of selecting the wrong test when selecting manually one the first, the second or the third test.

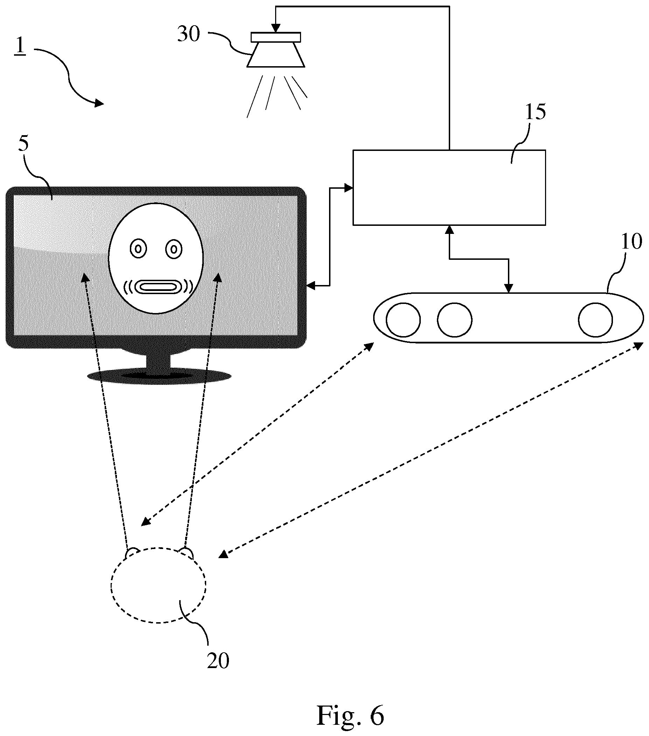

The invention also provides an apparatus for evaluating a risk of neurodevelopmental disorder with a child (20). An exemplary apparatus (1) is shown on Fig.6. It comprises a display screen (5), an eye-tracking device (10) and a controller (15). The controller (15) is configured for:

in the course of a first time period:

- displaying first information on the display screen (5) and meanwhile reading and recording eye position data from the eye-tracking device (10) while the child (20) is looking at the display screen (5), thereby obtaining first recorded movements of at least one eye of the child (20), and
- comparing data from the first recorded movement of the at least one eye of the child (20) with first pre-recorded reference data and calculating a first score in function of said comparison, and

in the course of a second time period:

- displaying second information on the display screen (5) and meanwhile reading and recording eye position data from the eye-tracking device (10) while the child (20) is looking at the display screen (5), thereby obtaining second recorded movements of at least one eye of the child (20), and
- comparing data from the second recorded movement of the at least one eye of the child (20) with second pre-recorded reference data and calculating a second score in function of said comparison,

wherein

- the second time period starts at least 24 hours later than the end of first time period,
- the second information is different from the first information, and
- the controller (15) is configured to calculate and deliver a composite score which is a function of at least the first score and the second score.

Such an apparatus (1) is adapted to perform a method according to the invention as previously described and therefore presents the corresponding advantages compared to conventional apparatus (1).

Preferably, the apparatus (1) is for evaluating a risk of an Autism Spectrum Disorder with a child (20). Preferably, the first time period is a within a period during which the child (20) has an age comprised between 4 and 10 months, preferably between 6 and 8 months; and the second time period is within a period during which the child (20) has an age comprised between 7 and 13 months, preferably between 9 and 11 months.

Preferably, the controller (15) is further configured for:

in the course of a third time period:

- displaying third information on the display screen (5) and meanwhile reading and recording eye position data from the eye-tracking device (10) while the child (20) is looking at the display screen (5), thereby obtaining third recorded movements of at least one eye of the child (20), and
- comparing data from the third recorded movement of the at least one eye of the child (20) with third pre-recorded reference data and calculating a third score in function of said comparison,

wherein

- the third time period starts at least 24 hours later than the end of second time period,
- the third information is different from the first and from the second information, and
- the controller (15) is configured to calculate and deliver a composite score which is a function of at least the first score, the second score and the third score.

Preferably, the third time period is a within a period during which the child (20) has an age comprised between 10 and 16 months, preferably between 12 and 14 months.

Preferably, the apparatus (1) further comprises a loudspeaker (30) and the controller (15) is configured for playing a sound via the loudspeaker (30) while displaying first and/or second and/or third information on the display screen (5).

It will be obvious that in any of the first, second and third tests, other parameters than fixations may be used to establish the respective first, second and third scores, such as a number of saccades, a pupil dilation or an amount of time spent looking at the display screen (5) for example.

The present invention has been described in terms of specific embodiments, which are illustrative of the invention and not to be construed as limiting. More generally, it will be appreciated by persons skilled in the art that the present invention is not limited by what has been particularly shown and/or described hereinabove.

Reference numerals in the claims do not limit their protective scope.

Use of the verbs "to comprise", "to include", "to be composed of", or any other variant, as well as their respective conjugations, does not exclude the presence of elements other than those stated.

Use of the article "a", "an" or "the" preceding an element does not exclude the presence of a plurality of such elements. The invention may also be described as follows: a method which performs at least two different tests on a given child at respectively at least two different ages of said child. In the course of a first test, a first information is displayed to the child and meanwhile a controller (15) acquires first data about eye movements of the child via an eye tracking device and determines a first score by comparison of said first data with first pre-recorded reference data. The same is performed in the course of a second test at a second age of said child with second information which is different from the first information, hence yielding a second score. A composite score is then calculated for said child in function of at least the first and the second scores. This composite score is a more reliable indication of a risk of neurodevelopmental disorder than indicators obtained with known methods.

**Claims**

1. Method for evaluating a risk of neurodevelopmental disorder with a child, the method comprising the steps of :

   - providing a machine comprising a display screen (5), an eye-tracking device (10), a controller (15) configured for displaying information on the display screen (5) and for reading and recording eye position data from the eye-tracking device (10);
   - having the child passing a first test in the course of a first time period, the first test comprising, in sequence:

     - having the child looking at the display screen (5) and directing the eye-tracking device (10) towards at least one eye of said child,
     - displaying first information on the display screen (5) and meanwhile reading and recording eye position data from the eye-tracking device (10), thereby obtaining first recorded movements of the at least one eye of the child,
     - comparing data from the first recorded movement of the at least one eye of the child with first pre-recorded reference data and calculating a first score in function of said comparison;
   - having the child passing at least a second test in the course of a second time period, the second test comprising, in sequence:

     - having the child looking at the display screen (5) and directing the eye-tracking device (10) towards at least one eye of said child,
     - displaying second information on the display screen (5) and meanwhile reading and recording eye position data from the eye-tracking device (10), thereby obtaining second recorded movements of the at least one eye of the child,
     - comparing data from the second recorded movement of the at least one eye of the child with second pre-recorded reference data and calculating a second score in function of said comparison;

   **characterized in that**

     - the second time period starts at least 24 hours later than the end of first time period,
     - the second information is different from the first information, and
     - the controller (15) calculates and delivers a composite score which is a function of at least the first score and the second score.

2. Method according to claim 1, **characterized in that** the neurodevelopmental disorder is an Autism Spectrum Disorder.

3. Method according to claim 1 or 2, **characterized in that**:

   - the first time period is a within a period during which the child has an age comprised between 4 and 10 months, preferably between 6 and 8 months; and **in that**
   - the second time period is within a period during which the child has an age comprised between 7 and 13 months, preferably between 9 and 11 months.

4. Method according to any of preceding claims, **characterized in that** it further comprises the steps of

   - having the child passing a third test in the course of a third time period, the third test comprising, in sequence:

     - having the child looking at the display screen (5) and directing the eye-tracking device (10) towards at least one eye of said child,
     - displaying third information on the display screen (5) and meanwhile reading and recording eye position data from the eye-tracking device (10), thereby obtaining third recorded movements of the at least one eye of the child,
     - comparing data from the third recorded movement of the at least one eye of the child with third pre-recorded reference data and calculating a third score in function of said comparison;

wherein

- the third time period starts at least 24 hours later than the end of second time period,
- the third information is different from the first and from the second information, and
- the controller (15) calculates and delivers a composite score which is a function of at least the first score, the second score and the third score.

5. Method according to claim 4, **characterized in that** the third time period is a within a period during which the child has an age comprised between 10 and 16 months, preferably between 12 and 14 months.

6. Method according to any of preceding claims, **characterized in that** it further comprises the steps of:

- providing a loudspeaker (30) and a controller (15) for playing a sound via the loudspeaker (30), and
- playing a sound via the loudspeaker (30) while displaying first and/or second and/or third information on the display screen (5).

7. Method according to any of preceding claims, **characterized in that** it further comprises the step of recording identification data of the child before starting the first test and the step of linking said data with the first score, the second score and the composite score obtained for said child.

8. Method according to any of preceding claims, **characterized in that** it further comprises the step of inputting the current age of the child before starting the first, the second or the third test and **in that** the controller (15) checks the inputted age of child and selects and executes one of the first, the second or the third test in function of the current age of the child.

9. Apparatus (1) for evaluating a risk of neurodevelopmental disorder with a child, said apparatus (1) comprising a display screen (5), an eye-tracking device (10) and a controller (15), said controller (15) being configured for:

in the course of a first time period:

- displaying first information on the display screen (5) and meanwhile reading and recording eye position data from the eye-tracking device (10) while the child is looking at the display screen (5), thereby obtaining first recorded movements of at least one eye of the child, and
- comparing data from the first recorded movement of the at least one eye of the child with first pre-recorded reference data and calculating a first score in function of said comparison, and

in the course of a second time period:

- displaying second information on the display screen (5) and meanwhile reading and recording eye position data from the eye-tracking device (10) while the child is looking at the display screen (5), thereby obtaining second recorded movements of at least one eye of the child, and
- comparing data from the second recorded movement of the at least one eye of the child with second pre-recorded reference data and calculating a second score in function of said comparison,

**characterized in that**

- the second time period starts at least 24 hours later than the end of first time period,
- the second information is different from the first information, and
- the controller (15) is configured to calculate and deliver a composite score which is a function of at least the first score and the second score.

10. Apparatus (1) according to claim 9, **characterized in that** the neurodevelopmental disorder is an Autism Spectrum Disorder.

11. Apparatus (1) according to claim 9 or 10, **characterized in that**:

- the first time period is a within a period during which the child has an age comprised between 4 and 10 months, preferably between 6 and 8 months; and **in that**

- the second time period is within a period during which the child has an age comprised between 7 and 13 months, preferably between 9 and 11 months.

12. Apparatus (1) according to any of claims 9 to 11, **characterized in that** the controller (15) is further configured for:

in the course of a third time period:

- displaying third information on the display screen (5) and meanwhile reading and recording eye position data from the eye-tracking device (10) while the child is looking at the display screen (5), thereby obtaining third recorded movements of at least one eye of the child, and
- comparing data from the third recorded movement of the at least one eye of the child with third pre-recorded reference data and calculating a third score in function of said comparison,

and **in that**

- the third time period starts at least 24 hours later than the end of second time period,
- the third information is different from the first and from the second information, and
- the controller (15) is configured to calculate and deliver a composite score which is a function of at least the first score, the second score and the third score.

13. Apparatus (1) according to claim 12, **characterized in that** the third time period is a within a period during which the child has an age comprised between 10 and 16 months, preferably between 12 and 14 months.

14. Apparatus (1) according to any of claims 9 to 12, **characterized in that** it further comprises a loudspeaker (30) and **in that** the controller (15) is configured for playing a sound via the loudspeaker (30) while displaying first and/or second and/or third information on the display screen (5).

Begin
Test 1 → Enter child's name,
age, and gender;
Enter current date

Eye-tracker
calibration

Display videos
(adult speaking;
pause; adult silent) ↔ Record eye-
tracker data

Analyse eye-
tracker data

End Test 1

Compare results with
1° pre-recorded
reference data;
Calculate Score1 → Individual file
- Score1

Minimum
24 hours

Begin
Test 2 → Load individual file

Eye-tracker
calibration

Display videos
(synchronous and
asynchronous ↔ Record eye-
tracker data

Analyse eye-
tracker data

End Test 2

Compare results with
2° pre-recorded
reference data;
Calculate Score2 → Individual file
- Score 1
- Score2

Fig. 1

Calculate composite
score based on Score1
and Score2

Fig. 2

Synchronous                                    Asynchronous

Fig. 3

Diverted gaze                                  Directed gaze

Fig. 4

## Fig. 5

Begin Test 1 → Enter child's name, age, and gender; Enter current date

↓

Eye-tracker calibration

↓

Display videos (adult speaking; pause; adult silent) ↔ Record eye-tracker data

↓ (from Display videos) End Test 1

Record eye-tracker data → Analyse eye-tracker data

↓

Compare results with 1° pre-recorded data; Calculate Score1 → Individual file - Score1

Minimum 24 hours

↓

Begin Test 2 → Load individual file

↓

Eye-tracker calibration

↓

Display videos (synchronous and asynchronous) ↔ Record eye-tracker data

↓ End Test 2

Record eye-tracker data → Analyse eye-tracker data

↓

Compare results with 2° pre-recorded data; Calculate Score2 → Individual file - Score 1 - Score2

Minimum 24 hours

↓

Begin Test 3 → Load individual file

↓

Eye-tracker calibration

↓

Display videos (diverted gaze; directed gaze) ↔ Record eye-tracker data

↓ End Test 3

Record eye-tracker data → Analyse eye-tracker data

↓

Compare results with 3° pre-recorded data; Calculate Score3 → Individual file - Score1 - Score 2 - Score 3

Calculate composite score based on Score1, Scor2, Score3

Fig. 6

Europäisches
Patentamt

European
Patent Office

Office européen
des brevets

# EUROPEAN SEARCH REPORT

**Application Number**

EP 18 18 2000

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/262613 A1 (KLIN AMI [US] ET AL) 15 September 2016 (2016-09-15) * abstract * * paragraph [0002] - paragraph [0015] * * paragraph [0077] - paragraph [0089] * * paragraph [0093] - paragraph [0097] * * paragraph [0117] * * paragraph [0129] - paragraph [0140] * * claims 1-4,11,16-19 * * figures 1,2,4,14,16A, 16B * | 1-14 | INV. G16H50/30 A61B5/16 A61B3/113 |
| X | US 2016/302713 A1 (MARUTA JUN [US] ET AL) 20 October 2016 (2016-10-20) * abstract * * paragraph [0022] - paragraph [0032] * * paragraph [0082] - paragraph [0088] * * claims 1,6,7,12,13,16,18 * * figures 1-3 * | 1-14 | |
| A | US 2016/022136 A1 (ETTENHOFER MARK L [US] ET AL) 28 January 2016 (2016-01-28) * the whole document * | 1-14 | TECHNICAL FIELDS SEARCHED (IPC) G16H A61B |

The present search report has been drawn up for all claims

| Place of search | Date of completion of the search | Examiner |
|---|---|---|
| Munich | 10 December 2018 | Hilbig, Matthias |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

............................................................

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 18 18 2000

This annex lists the patent family members relating to the patent documents cited in the above-mentioned European search report.
The members are as contained in the European Patent Office EDP file on
The European Patent Office is in no way liable for these particulars which are merely given for the purpose of information.

10-12-2018

| Patent document cited in search report | | | Publication date | Patent family member(s) | | | Publication date |
|---|---|---|---|---|---|---|---|
| US 2016262613 | A1 | | 15-09-2016 | AU | 2014334870 | A1 | 12-05-2016 |
| | | | | CA | 2927583 | A1 | 23-04-2015 |
| | | | | CN | 105873515 | A | 17-08-2016 |
| | | | | EP | 3057508 | A1 | 24-08-2016 |
| | | | | JP | 2016537067 | A | 01-12-2016 |
| | | | | KR | 20160106552 | A | 12-09-2016 |
| | | | | SG | 11201602944Y | A | 30-05-2016 |
| | | | | US | 2016262613 | A1 | 15-09-2016 |
| | | | | WO | 2015057321 | A1 | 23-04-2015 |
| US 2016302713 | A1 | | 20-10-2016 | US | 2016302713 | A1 | 20-10-2016 |
| | | | | WO | 2016168724 | A1 | 20-10-2016 |
| US 2016022136 | A1 | | 28-01-2016 | AU | 2014249335 | A1 | 08-10-2015 |
| | | | | CA | 2905760 | A1 | 09-10-2014 |
| | | | | EP | 2991541 | A1 | 09-03-2016 |
| | | | | US | 2016022136 | A1 | 28-01-2016 |
| | | | | WO | 2014164453 | A1 | 09-10-2014 |

EPO FORM P0459

For more details about this annex : see Official Journal of the European Patent Office, No. 12/82

**EP 3 591 664 A1**

**Patent documents cited in the description**

- WO 2015057321 A
- WO 2014164858 A